# EUROPEAN PATENT APPLICATION

(11) **EP 2 944 321 A1**
(43) Date of publication of application: **18.11.2015**
(21) Application number: 14167898.7
(22) Date of filing: 12.05.2014
(51) Int. Cl.: A61K 39/12

(54) **Immunogenic peptides of EBOLA and applications thereof**

(71) Applicant: Institut De Recherche Pour Le Développement (IRD), 13572 Marseille Cedex 02 (FR)
(72) Inventor: Becquart, Pierre Jean, 34270 Lauret (FR); Leroy, Eric Maurice, BP769 Franceville (GA)
(74) Representative: Cabinet Armengaud Aîné

(57) **Abstract**

The invention relates to an immunogenic peptide, characterized in that it consists of 15 to 25 contiguous amino acids of an EBOV protein and that it comprises a sequence of 15 aminoacids selected in the group comprising sequences SEQ ID N°1 - 61.

## Description

The invention relates to immunogenic peptides of EBOLA. It also relates to the application thereof in a diagnostic test and for preparing vaccine compositions.

Ebola virus (EBOV), a member of the *Filoviridae* family, is a highly virulent pathogen for humans and nonhuman primates. EBOV is a filamentous enveloped virus containing a negative-strand RNA genome of about 19 kb. The EBOV genome codes for eight major subgenomic mRNAs that sequentially encode seven structural proteins, namely a nucleoprotein (NP), two virion proteins (VP35 and VP40), a surface glycoprotein (GP), two additional viral proteins (VP30 and VP24), an RNA-dependent RNA polymerase (L), and a non-structural soluble protein (sGP) [1].

After an incubation period ranging from 2 to 21 days (mean 4-9 days), EBOV causes severe hemorrhagic fever that is fatal in nearly 90% of cases within 7-11 days. EBOV has caused several outbreaks in Gabon, Democratic Republic of the Congo (DRC) and Republic of the Congo (RC).

There is currently no vaccine and no specific treatment. Fatal EBOV infection is characterized by a defective innate immune response, leading to uncontrolled release of inflammatory mediators and chemokines in the late stage of the disease, and correlates with the collapse of adaptive immunity with massive T and B lymphocyte apoptosis.

However, lethally infected model mice generated a functional CD8+ T cell response despite significant T cell apoptosis.

Survivors and asymptomatic subjects develop an early and moderate inflammatory response together with an effective adaptive response.

Defective adaptive immunity observed in fatal cases is associated with an impaired humoral response, as EBOV-specific IgG and IgM are barely detectable before death.

In contrast, recovery is associated with early, increasing levels of long-lasting EBOV-specific IgG, followed by viral antigen clearance.

Moderate amounts of EBOV-specific IgG are also detected about 3 weeks after infection in asymptomatic patients. A strong early humoral response may thus play a major role in survival.

Little is known of human antibody targets in EBOV infection. Western Blot analysis has shown that IgG antibodies in sera from survivors of symptomatic infection and from asymptomatic subjects are mainly directed against NP and VP40, and, in a minority of cases, against VP35. Similar results have been reported in seropositive individuals who have never had clinical signs of hemorrhagic fever or who live in non-epidemic areas. Antibody phage display libraries constructed from RNA derived from two survivors of the 1995 EBOV outbreak in Kikwit (DRC) also showed the presence of antibodies reacting with NP, GP and sGP. Little further information is available on human B-cell epitopes of EBOV proteins and therefore specific tools to diagnostic an infection and/or develop means to prevent or treat an EBOV infection today remains a challenge to date. The inventors' works in that field focussed on the characterization of the humoral response in individuals infected by EBOV by identifying the epitopes from VP35, VP40, NP and GP recognized by IgG anti-EBOV of sera of these individuals.

The object of the invention is then to provide isolated immunogenic peptide of EBOV.

Another object of the invention is to provide compared to the present tests a more specific and sensitive serological test for EBOLA to detect anti-EBOV antibodies in humans.

The object of the invention is also to provide a vaccine composition against EBOV.

The invention relates to an isolated immunogenic peptide having at least one sequence of 5 -35 contiguous amino acids of an EBOV protein, said at least one sequence being selected from the group comprising SEQ ID N°1-61 or being a fragment thereof.

In a first group, said EBOV protein is VP40. Preferred sequences are selected from the group comprising sequences SEQ ID N°1-13.

In a second group, said EBOV protein is GP. Preferred sequences are selected from the group comprising sequences SEQ ID N°14-39.

In a third group, said EBOV protein is VP35. Preferred sequences are selected from the group comprising sequences SEQ ID N°40-53.

In a fourth group, said EBOV protein is NP. Preferred sequences are selected from the group comprising sequences SEQ ID N°54-61.

In a preferred embodiment, the immunogenic peptide has a sequence corresponding to one of sequences SEQ ID N°1-61.

In another preferred embodiment, the immunogenic peptide comprises at least 2 sequences from the group consisting of sequences SEQ ID N°1-61, particularly 2-5 sequences from said group.

According to a further embodiment, the above defined immunogenic peptides of the invention comprise one or more terminal aminoacid heterologous to EBOV.

The invention also relates to an immunogenic preparation, comprising a combination of immunogenic peptides such as above defined,

The above defined peptides are linear peptides. They are specifically targeted by the sera of patients. As shown in the examples, humoral response of survivors of EBOV outbreaks mainly targeted GP peptides, whereas sera from EBOV-seropositive asymptomatic subjects reacted strongly with VP40 peptides.

The immunogenic peptides are B-cell epitopes of high interest for the development of new therapeutic and diagnostic tools. Particularly, the immunopeptides and immunogenic preparations are useful to stimulate an immune response directed against EBOV, and therefore to prepare vaccinal compositions.

They are also useful as diagnostic tools and for the serological detection *in vitro* of EBOV.

The invention thus also relates to a kit for the specific diagnosis of EBOV in a patient, comprising at least one immunogenic peptide such as above defined.

It also relates to a diagnostic kit wherein the peptide is fixed on a support.

Advantageously, said diagnostic kit further comprises a detectable antibody directed against one or more classes of human IgG. Said detectable antibody is for example fluorescent, bound to an enzyme and optionally comprises a substrate of the enzyme allowing a visual detection.

According to another aspect, the invention also relates to a method for serological detection of EBOV in a biological sample, comprising
- contacting the biological sample with an immunogenic peptide or an immune preparation such as above defined
- detecting the binding of the peptide with antibodies of the sample, for example by ELISA test.

The ELISA test is for example performed as disclosed by Ksiazek *et al* (2).

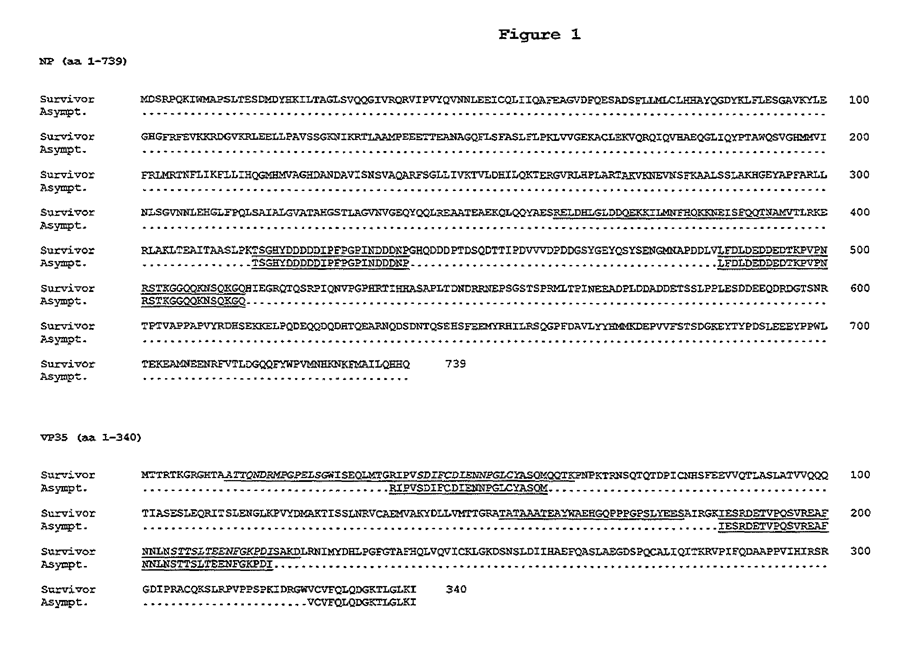

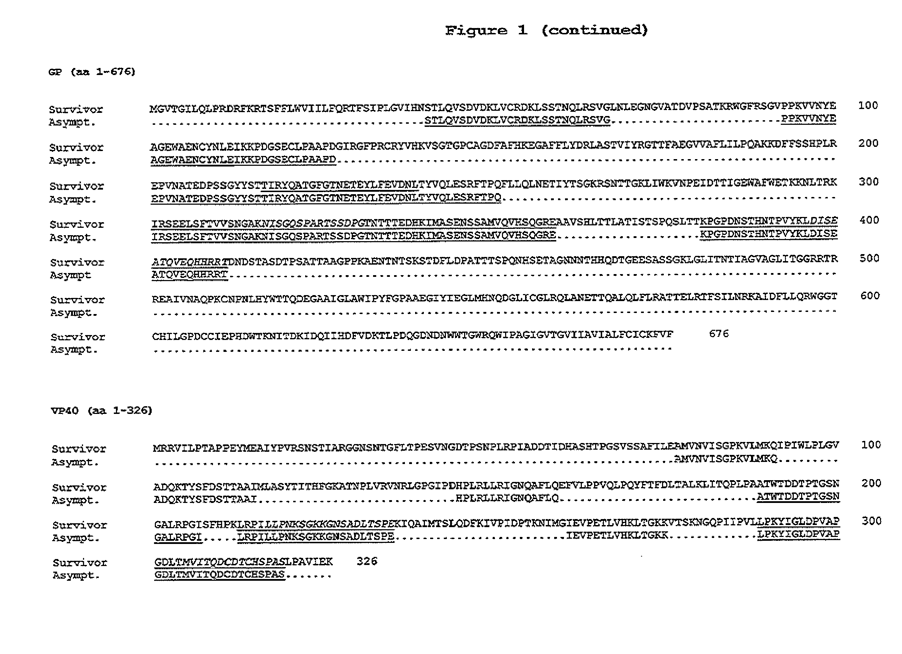

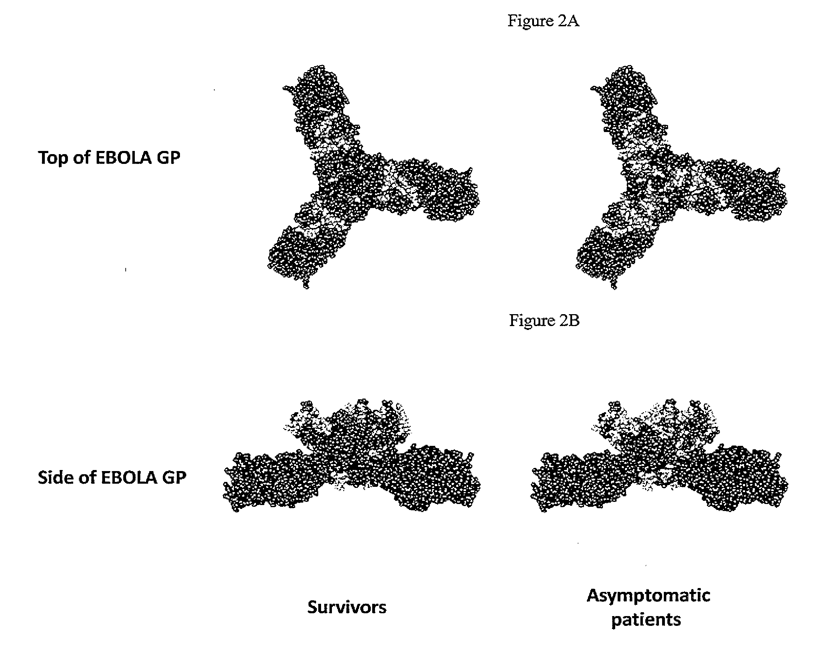

Other characteristics and advantages of the invention are given in the following examples wherein it is referred to Figures 1 and 2, wherein:
- Figure 1 gives the amino acid sequences of
   - GP (positions 1-676)
   - VP40 (positions 1-326),
   - NP (positions 1-739), and
   - VP35 (positions 1-340),
with identification in bold characters of the epitopes having reacted with sera collected from survivors of the three outbreaks in Gabon, 7 days after symptom onset, and from asymptomatic individuals (Asympt.) who had never had clinical signs of hemorrhagic fever or who lived in non epidemic areas.

The sequences for the survivors and the asymptomatic individuals are, respectively, as follows:
SEQ ID N°62 and SEQ ID N°63 with respect to GP
SEQ ID N°64 and SEQ ID N°65 with respect to VP40
SEQ ID N°66 and SEQ ID N°67 with respect to NP
SEQ ID N°68 and SEQ ID N°69 with respect to VP35.

Epitopes reacting with sera from survivors of the three outbreaks, collected 7 or 11 years after recovery,are indicated in italics. The principal immunogenic regions are underlined.
- Figure 2, the crystal structure of the trimeric prefusion EBOV GP viewed from the top and side. The three monomers in the complex are coloured in grey. Immunodominant regions identified in GP are indicated in white for survivors and asymptomatic patients..Protein Data Bank file number: 3CSY.

### Materials and Methods

### B cell epitope mapping

Identify linear epitopes and characterize immunodominant sites in EBOV. Epitope mapping was applied to four EBOV proteins representing the main targets of the humoral response, namely VP35, VP40, NP and GP.

Five hundred and six peptides (consecutive 15-mers with an overlap of 11 aminoacids (aa) were synthesized.

The peptides spanned the 676 aa of GP (162 peptides), the 739 aa of NP (182 peptides), the 326 aa of VP40 (79 peptides) and the 340 aa of VP35 (83 peptides), as deduced from the EBOV genome isolated during the 1995 outbreak in Kikwit, DRC.

Each lyophilized peptide was solubilized in dimethyl formamide and adjusted to a final concentration of 5 mg/mL with phosphate buffered saline (PBS) before use (Sigma, France).

### Ebola patient sera

Serum samples from EBOV-infected patients were used. The samples were collected during the outbreaks in the villages of Mayibout, Booué (1996) and Mekambo (2001) in Gabon.

The first outbreak hit the village of Mayibout, located in north-eastern Gabon, from January to February 1996, causing 10 non-fatal clinical cases and 21 deaths.

The second outbreak caused 45 deaths among 60 cases between October 1996 and March 1997 in the Booué area, 150 km southwest of Mayibout. The infection spread to several villages around Booué.

The third outbreak occurred between October 2001 and May 2002 in the Mekambo area of Gabon and the Mbomo area of Republic of Congo, 150 km east of Mayibout.

A total of 207 human cases (58 survivors and 149 deaths) were recorded. A total of 298 human cases and 215 deaths were reported during these three outbreaks.

Sera from two groups of survivors were used to map B cell epitopes.

The first consisted of 15 stored sera collected during the three outbreaks from survivors of laboratory-confirmed infection, seven days after the end of symptoms (Survivor group #1).

The second (Survivor group #2) consisted of sera collected in 2008 from a survivor of the 1996 Mayibout outbreak, two survivors of the 1996-1997 Booué outbreak and three survivors of the 2001 Mekambo outbreak.

Early humoral responses to EBOV were analyzed in the first survivor group, and late humoral responses were analyzed in the second group.

All the individuals were adults (>16 years), except for patient #19 who was 7 years old and patients #1 and #2 who were 15 years old.

21 sera from EBOV IgG-seropositive adults who had never had clinical signs of hemorrhagic fever or who lived in non-epidemic areas were also screened. These individuals, selected randomly in various parts of Gabon, had tested positive for anti-EBOV antibodies in an indirect IgG ELISA and by Western Blot. They lived in rural villages with fewer than 300 inhabitants, located in the nine administrative regions of Gabon.

As negative controls, 20 individuals were randomly selected from Gabon. They had been tested negative for EBOV by indirect ELISA and Western Blot methods.

Sera from each group were pooled for the first screen with all the synthetic peptides. The first two pools consisted of sera from eight and seven survivors collected 7 days after the end of symptoms (Survivor group #1). The third pool consisted of six stored sera from survivors collected in 2008 (Survivor group #2). The last three pools each consisted of sera from seven asymptomatic individuals. Two pools of sera from ten EBOV-naive individuals were used as negative controls.

### Ethics statement

The study received the approval of the Gabonese Ministry of Health (research authorization Nb. 00093, March 15, 2005). Samples from the present study came from two previous sero-surveys conducted by a multidisciplinary team, including a doctor from the Gabonese Ministry of Health, a nurse, an epidemiologist, a virologist, a veterinarian and laboratory technicians. The studies were approved by the traditional chief of each village, and written informed consent was obtained from each survivor and asymptomatic patient. The studies were described orally, and volunteers gave their signed informed consent to be enrolled in the studies and for their blood samples to be used for future research studies. In the case of children, the parents' written consent was obtained for their clinical records and samples to be used for future research studies.

### Indirect ELISA for peptide epitope identification

Briefly, plates (Nunc-immuno Maxisorb^{™} plates, Nunc, Denmark) were coated with 5 µg/mL single synthetic peptide in 100 µL/well PBS overnight at +4°C. The wells were then washed three times with PBS containing 0.1% (vol/vol) Tween 20 (Sigma) (PBS-T). To block non-specific binding, the wells were incubated with 200 µL of PBS-T containing 5% (weight/vol) non-fat milk (Becton Dickinson) (mPBS-T) for 1 hour at 37°C. Several serum dilutions (1:100 to 1:1600) were tested for each peptide. The 1:400 dilution provided the best compromise between sensitivity and specificity in the ELISA. Therefore, after the washing step described above, 1:400-diluted sera in mPBS-T were incubated in the wells overnight at +4°C. The wells were then washed three times with PBS-T and 100 µL of goat anti-human IgG peroxidase-labeled antibody (Sigma) was added at 1:2000 dilution in mPBS-T for 1 hour at room temperature. After a final washing step with PBS-T, 100 µL of a 1:1 solution of tetramethylbenzydine (TMB, Thermo Electron Corporation) and hydrogen peroxide (Dynex Technologies, France) was added to each well for 10 minutes at room temperature. Optical density (OD) was then measured at 450 nm with an ELISA plate reader (Bio-Rad PR3100).

All **the** peptides were tested in triplicate against the positive and negative serum pools as described above. The mean OD obtained with the negative serum pool, plus three times the standard deviation, was used as the cut-off value. The reactive peptides identified in the first screen were then tested in triplicate with individual sera from the 21 survivors, the 21 positive asymptomatic seropositive subjects, and the 20 EBOV-naive individuals. The same cut-off as above was used for each reactive peptide selected in the first screen. Final results are reported as mean OD.

### Results

### Epitope mapping of VP35, VP40, NP and GP

### 1. Identification and location of reactive peptides in survivors

In the first screen, the two pools of sera from survivors collected 7 days after the end of symptoms (Survivor group #1) and from survivors collected in 2008 (Survivor group #2) reacted with 14 VP35 peptides, 8 NP peptides, 7 VP40 peptides, and 19 GP peptides.

In the second screen, sera from the 21 survivors were tested with these reactive peptides.

The sequences of these peptides and the mean ODs are summarized in Tables 1A, 1B, 1C and 1D given hereinafter. The humoral response was mainly directed against GP. None of the peptides was recognized by all of the survivor group #1 and #2 sera, but GP peptide 78, NP peptide 105 and VP40 peptides 73 and 77 reacted with more than half of the group #1 sera. These peptides composed several linear regions which were similar during the three EBOV outbreaks that occurred in Gabon between 1996 and 2001.

10 immunogenic regions were identified in the four proteins tested (Figure 2). The immunodominant epitopes recognized by anti-EBOV IgG were located in three principal regions ofNP and GP at aa positions 361-395, 417-439, 485-515 and 216-239, 301-359, 381-411 respectively. Two antigenic regions were mapped in VP40 (213-235 and 289-319) and VP35 (13-63 and 153-223). Sera of survivor groups #1 and #2 reacted with GP, VP40, NP and VP35 peptides in 71%, 100%, 38% and 38% of cases, respectively. The crystal structure of the trimeric prefusion EBOV GP complexed with a neutralizing antibody from a human survivor was used to locate GP immunodominant regions with the assistance of Cn3D 4.3 software (National Center for Biotechnology Information). In the crystal structure of GP lacking the mucin-like domain, survivors' IgG was mainly directed against epitopes located on the inner and outer surfaces of the chalice bowl formed by three GP₁ viral attachment subunits, cradled by the GP₂ fusion subunits (Figure 2).

### 2. Identification and location of reactive peptides in asymptomatic subjects

Peptide ELISA was also used to screen sera from anti-EBOV IgG+ individuals who had never had clinical signs of hemorrhagic fever or who lived in non-epidemic areas, and in whom had previously detected both EBOV-specific IgG and IFN-γ-producing CD8+ T cell responses. In the first screen, the three pools, each containing sera from seven asymptomatic individuals, reacted with seven peptides located in VP35, 11 in VP40, four in NP and 22 in GP. In the second screen, sera from the 21 positive asymptomatic seropositive subjects were tested individually with these reactive peptides. The sequences of these peptides and their adjusted ODs are summarized in Tables 1A, 1B, 1C and 1D given hereinafter.

Said Tables give results concerning mean OD values at 1:400 serum dilution in patients infected with EBOV during three outbreaks in Gabon, 7 days after symptom onset (Day 7, early humoral response) and 7 or 11 years later (2008, late humoral response), and also in anti-EBOV IgG+ asymptomatic individuals who had never had clinical signs of hemorrhagic fever or who lived in non epidemic areas*. ODs are color-coded to indicate their intensity (yellow to red). The different colored regions in the proteins indicate the immunodominant domains identified here.
*Blank cells indicate that the mean OD was lower than the cut-off. OD: Optical Density. The absorbance cut-off used to identify reactive epitopes is described in the Methodology section.

The peptides identified in Table 1A have the following SEQ ID N° and positions on the EBOV proteins:

**GP**

| **Peptide #** | **Peptides (aa)** | **Position** | **SEQ ID N°** |
|---|---|---|---|
| 11 | STLQVSDVDKLVCRD | 41-55 | 14 |
| 14 | CRDKLSSTNQLRSVG | 52-66 | 15 |
| 24 | PPKVVNYEAGEWAEN | 93-107 | 16 |
| 29 | IKKPDGSECLPAAPD | 112-126 | 17 |
| 51 | EPVNATEDPSSGYYS | 201-215 | 18 |
| 55 | TIRYQATGFGTNETE | 217-231 | 19 |
| 56 | QATGFGTNETEYLFE | 221-235 | 20 |
| 57 | FGTNETEYLFEVDNL | 225-239 | 21 |
| 60 | DNLTYVQLESRFTPQ | 236-250 | 22 |
| 76 | IRSEELSFTVVSNGA | 301-305 | 24 |
| 78 | TVVSNGAKNISGQSP | 309-323 | 25 |
| 80 | NISGQSPARTSSDPG | 317-331 | 26 |
| 81 | QSPARTSSDPGTNTT | 321-335 | 27 |
| 82 | RTSSDPGTNTTTEDH | 325-339 | 28 |
| 83 | DPGTNTTTEDHKIMA | 329-343 | 29 |
| 84 | NTTTEDHKIMASENS | 333-347 | 30 |
| 85 | EDHKIMASENSSAMV | 337-351 | 31 |
| 86 | IMASENSSAMVQVHS | 341-355 | 32 |
| 87 | ENSSAMVQVHSQGRE | 345-359 | 33 |
| 96 | KPGPDNSTHNTPVYK | 381-395 | 34 |
| 97 | DNSTHNTPVYKLDIS | 385-399 | 35 |
| 98 | HNTPVYKLDISEATQ | 389-403 | 36 |
| 99 | VYKLDISEATQVEQH | 393-407 | 37 |
| 100 | DISEATQVEQHHRRT | 397-411 | 38 |
| 118 | TGEESASSGKLGLIT | 469-383 | 39 |

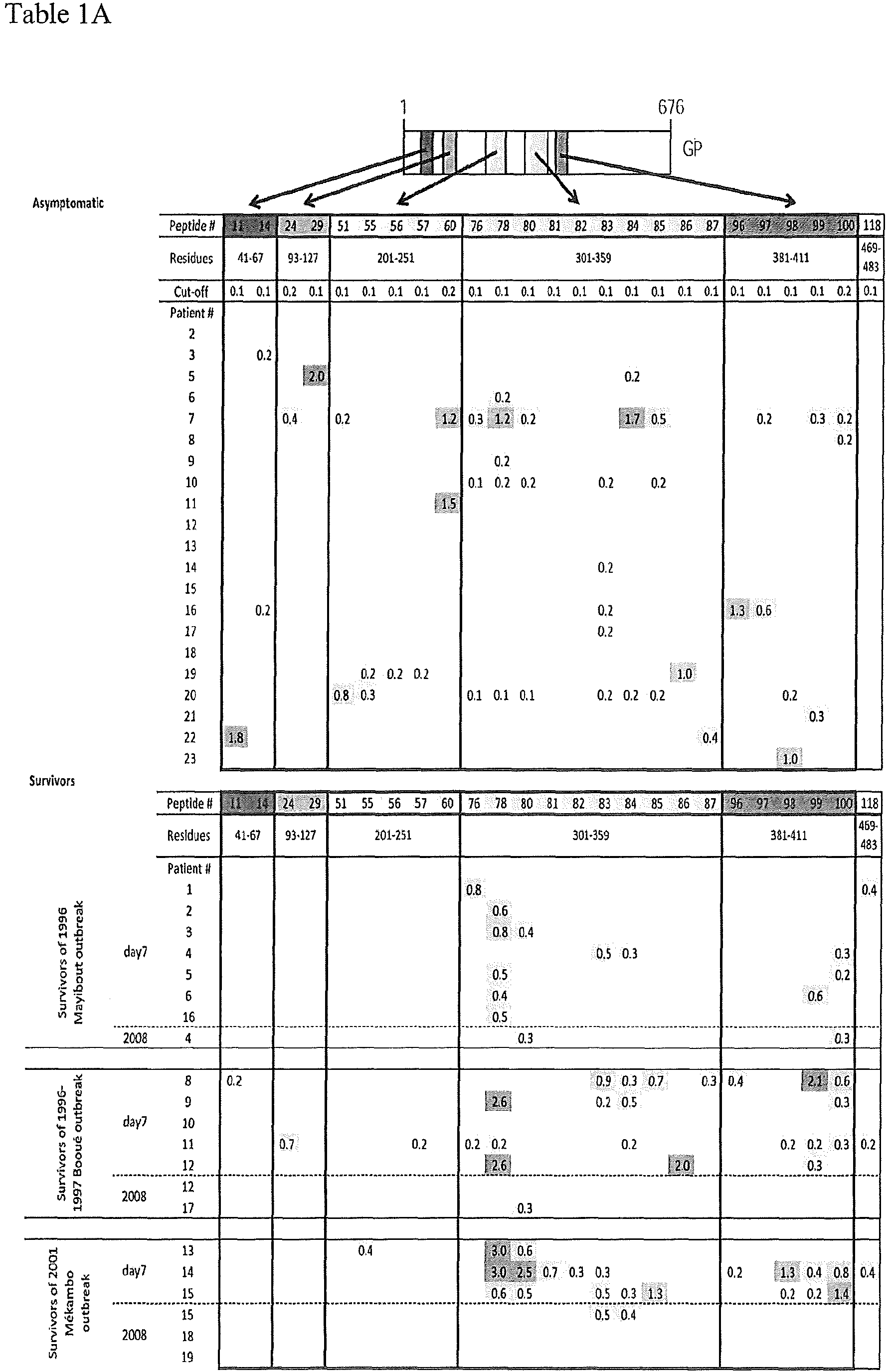

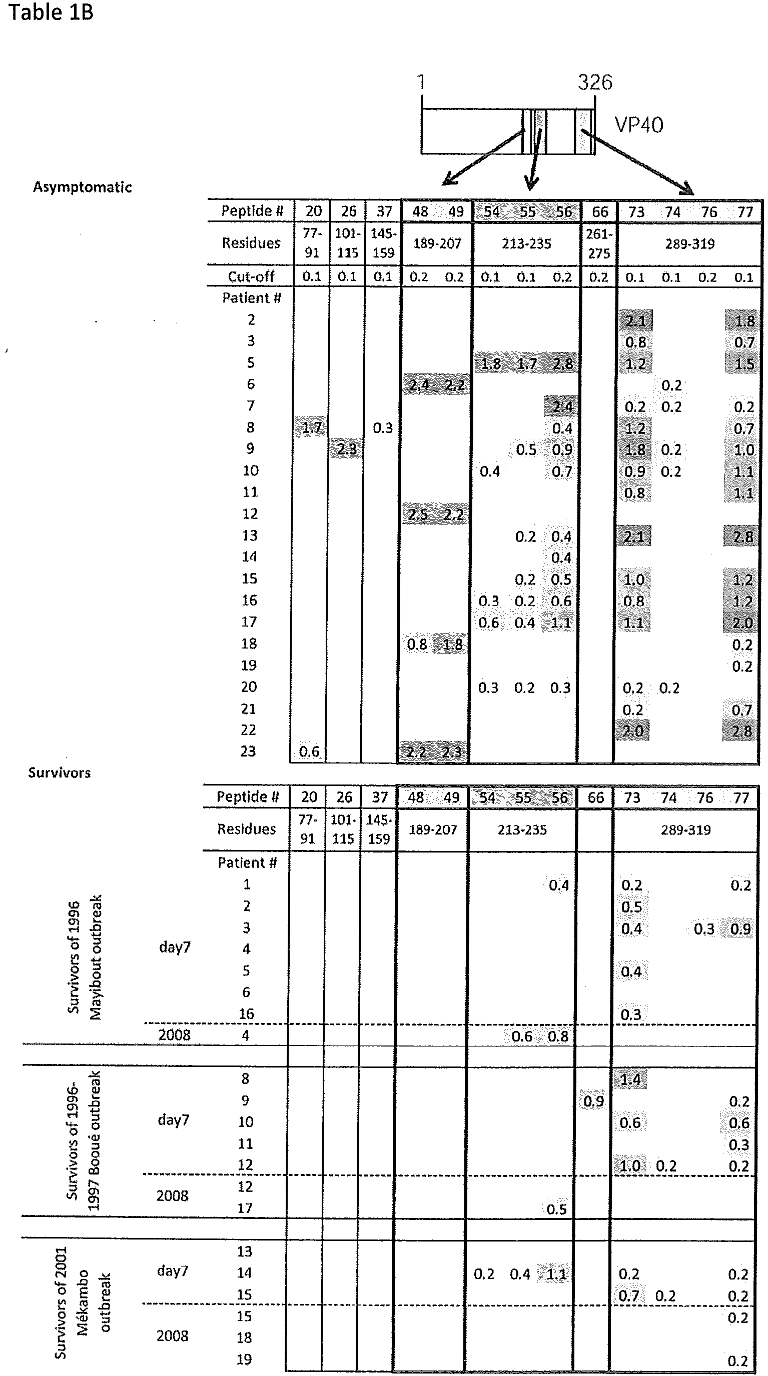

The peptides identified in Table 1B have the following SEQ ID N° and positions on the EBOV proteins:

**Protein VP40**

| **Peptide #** | **Peptides (aa)** | **Position** | **SEQ ID N°** |
|---|---|---|---|
| 20 | AMVNVISGPKVLMKQ | 77-91 | 1 |
| 26 | ADQKTYSFDSTTAAI | 100-114 | 2 |
| 37 | HPLRLLRIGNQAFLQ | 144-158 | 3 |
| 48 | ATWTDDTPTGSNGAL | 188-202 | 4 |
| 49 | DDTPTGSNGALRPGI | 192-206 | 5 |
| 54 | LRPILLPNKSGKKGN | 213-227 | 6 |
| 55 | LLPNKSGKKGNSADL | 217-231 | 7 |
| 56 | KSGKKGNSADLTSPE | 221-235 | 8 |
| 66 | IEVPETLVHKLTGKK | 261-275 | 9 |
| 73 | LPKYIGLDPVAPGDL | 289-303 | 10 |
| 74 | IGLDPVAPGDLTMVI | 293-307 | 11 |
| 76 | GDLTMVITQDCDTCH | 301-315 | 12 |
| 77 | MVITQDCDTCHSPAS | 305-319 | 13 |

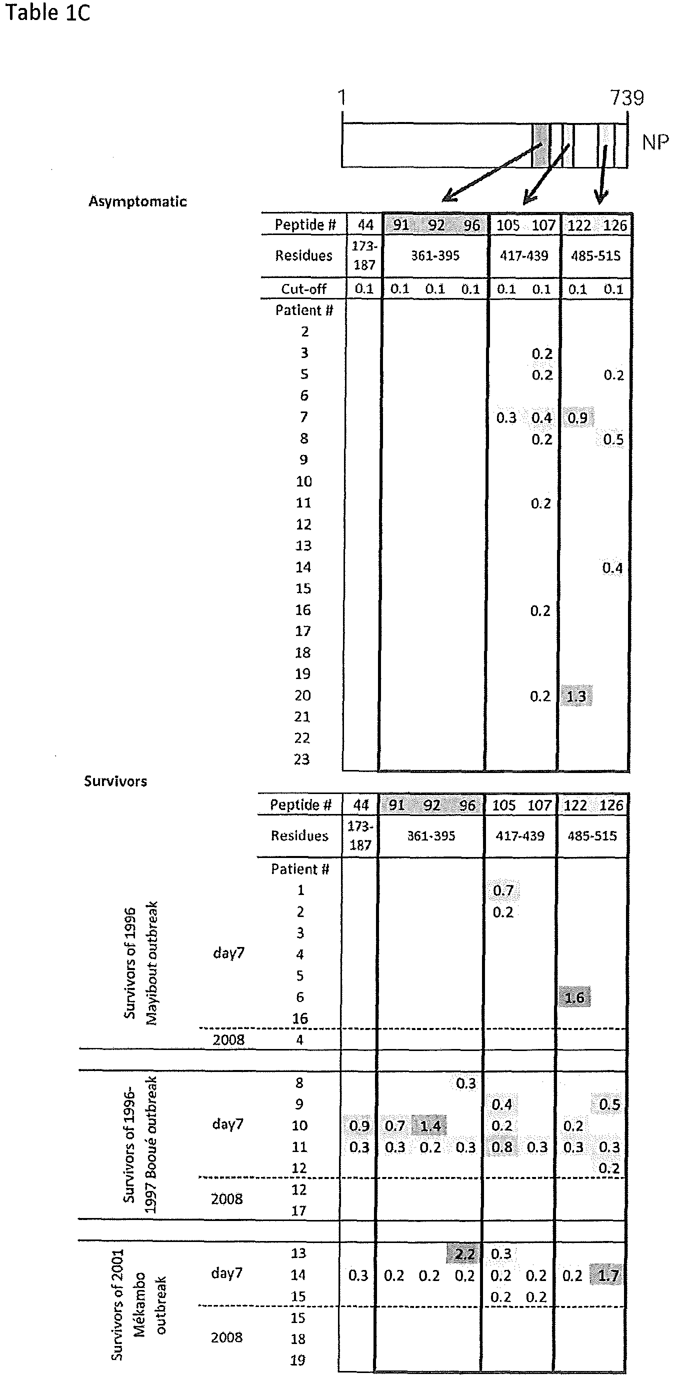

The peptides identified in Table 1C have the following SEQ ID N° and positions on the EBOV proteins:

**Protein NP**

| **Peptide #** | **Peptides (aa)** | **Position** | **SEQ ID N°** |
|---|---|---|---|
| 44 | QRQIQVHAEQGLIQY | 173-187 | 54 |
| 91 | RELDHLGLDDQEKKI | 361-375 | 55 |
| 92 | HLGLDDQEKKILMNF | 365-379 | 56 |
| 96 | QKKNEISFQQTNAMV | 381-395 | 57 |
| 105 | TSGHYDDDDDIPFPG | 417-431 | 58 |
| 107 | DDIPFPGPINDDDNP | 425-439 | 59 |
| 122 | LFDLDEDDEDTKPVP | 485-499 | 60 |
| 126 | RSTKGGQQKNSQKGQ | 501-515 | 61 |

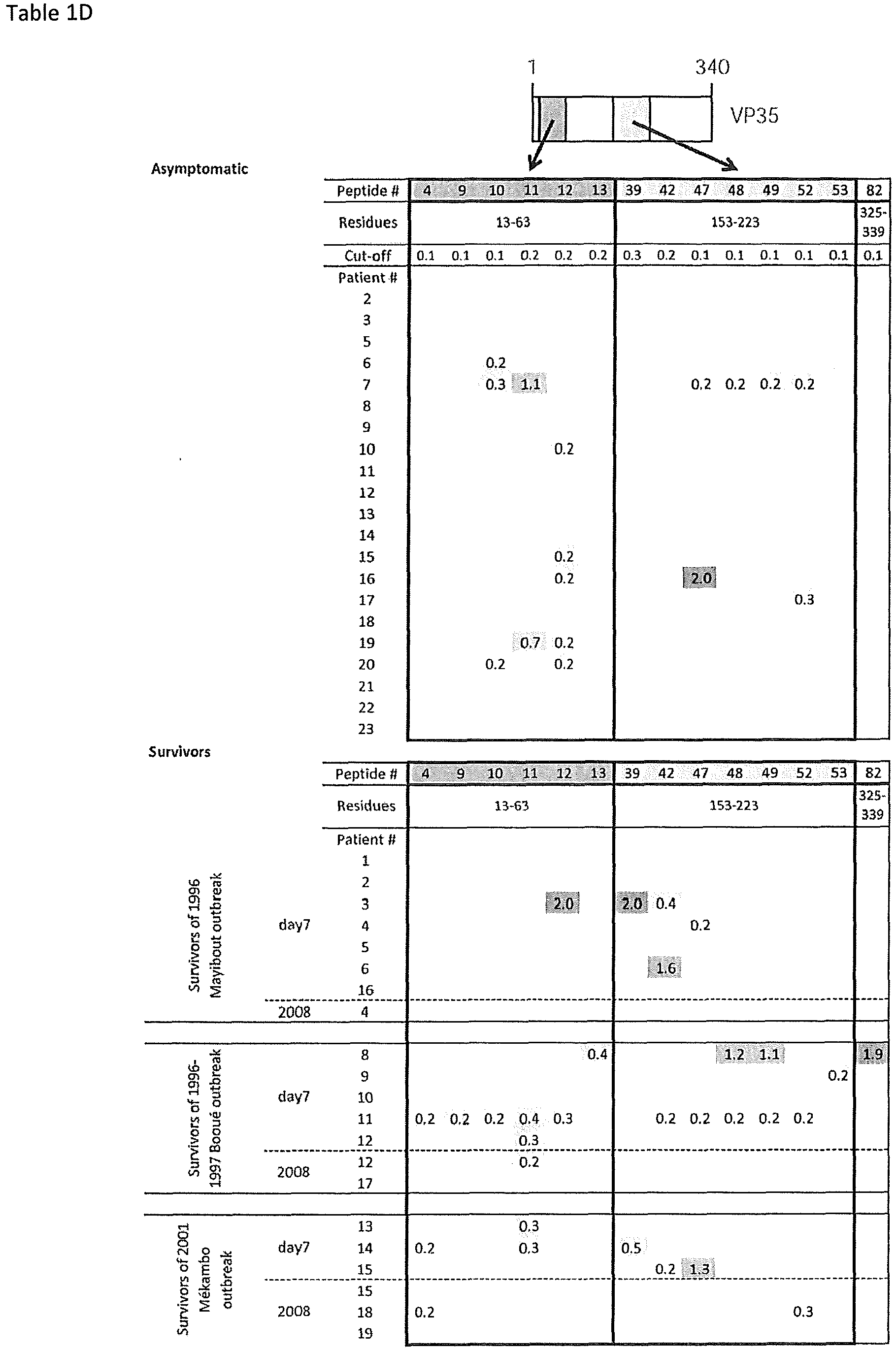

The peptides identified in Table 1D have the following SEQ ID N° and positions on the EBOV proteins:

**Protein VP35**

| **Peptide #** | **Peptides (aa)** | **Position** | **SEQ ID N°** |
|---|---|---|---|
| 4 | ATTQNDRMPGPELSG | 13-27 | 40 |
| 9 | LMTGRIPVSDIFCDI | 33-47 | 41 |
| 10 | RIPVSDIFCDIENNP | 37-51 | 42 |
| 11 | SDIFCDIENNPGLCY | 41-55 | 43 |
| 12 | CDIENNPGLCYASQM | 45-59 | 44 |
| 13 | NNPGLCYASQMQQTK | 49-63 | 45 |
| 39 | TATAAATEAYWAEHG | 153-167 | 46 |
| 42 | EHGQPPPGPSLYEES | 165-179 | 47 |
| 47 | IESRDETVPQSVREA | 185-199 | 48 |
| 48 | DETVPQSVREAFNNL | 189-203 | 49 |
| 49 | DETVPQSVREAFNNL | 193-207 | 50 |
| 52 | STTSLTEENFGKPDI | 205-219 | 51 |
| 53 | LTEENFGKPDISAKD | 219-223 | 52 |
| 82 | VCVFQLQDGKTLGLK | 325-339 | 53 |

Most of the reactive peptides were located in 11 linear immunogenic regions at aa positions 37-59 and 153-219 for VP35, 417-439 and 485-515 for NP, 189-235 and 289-319 for VP40 and 41-67, 93-127, 201-251, 301-359 and 381-411 for GP (Figure 1). GP, VP40, NP and VP35 peptides reacted respectively with 81%, 76%, 52% and 57% of sera from asymptomatic individuals. In these subjects the main target of the anti-EBOV IgG response was VP40 (Table 1B). As in survivors, none of the peptides reacted with all the sera, but VP40 peptides 73 and 77 reacted with more than 70% of the sera from the 21 individuals (n=15 and n=16, respectively). The positions of the immunodominant regions in a three-dimensional structural projection of GP were located as described above. It was found that the epitopes recognized by these asymptomatic subjects' sera were located in the chalice bowl formed by the three GP₁ subunits (Figure 2).

### 3. Comparison between early and late responses in survivors

Although the number of survivors of the 1996 and 2001 outbreaks available for sampling in 2008 was small (survivors #4, #12 and #15), the data suggests that the number of B-cell epitopes is far lower in sera collected seven to twelve years after infection than in sera collected during the acute phase (Tables 1A to 1D). Indeed, EBOV peptide-reactive IgG was undetectable in sera collected more than 11 years after infection (Survivor group #2), compared to sera from individuals in early convalescence (Survivor group #1) (n=23; 74.2%).

### 4. Comparison between symptomatic and asymptomatic subjects

The panel of peptides recognized by sera from asymptomatic subjects was similar to that recognized by sera from the two groups of survivors (Figure 1). Proportionally, the IgG response in survivors was mainly directed against GP, while in asymptomatic subjects it was mainly directed against VP40. In addition, more VP40 immunogenic regions were found in asymptomatic patients than in survivors (7 *versus* 3). Interestingly, in the asymptomatic subjects the longest immunodominant domains were identified in GP₁, and the crystal structure of GP revealed that these regions covered a larger surface area of the chalice bowl formed by the three GP₁ subunits (Figure 2).

### Discussion

As shown by the above results, epitopes were identified in GP, NP, VP40 and VP35, the main antibody targets in EBOV- infected patients. Sera from asymptomatic subjects and survivors were tested against a set of overlapping 15-residue synthetic peptides that spanned the four proteins.

It was found that antisera from asymptomatic patients targeted and reacted strongly with a larger number of peptides derived from VP40 than from GP, NP or VP35. In addition, a previous Western blot analysis of sera collected from asymptomatic patients revealed that IgG responses were directed against both VP40 and NP.

The structural protein VP40 is the most abundant protein in EBOV particles, representing about 40% by molecular weight.

The matrix proteins VP40 and VP24 are linked to the ribonucleoprotein complex at the inner surface of the lipid bilayer of the viral envelope (which is derived from the host cell), ensuring the structural integrity of the particle. VP40 plays a crucial role in virus assembly and budding. This matrix protein is able to produce virus-like particles from cells in the absence of other viral proteins. first elucidated its crystal structure and suggested that the protein consists of two distinct N- and C-terminal domains (NTD and CTD) connected by a flexible linker, both essential for trafficking to and interaction with the membrane.

More recent crystal structures reveal that VP40 is a dimer, assembling end-to-end into filaments, through homologous interactions between their CTDs.

The inventors found that serum from asymptomatic individuals reacted strongly with three immunodominant domains located in the CTD of VP40 (aa residues 189-207, 213-235 and 289-319), while serum from survivors reacted less strongly and with fewer peptides.

The first region, targeted only by IgG from asymptomatic patients, is located in the flexible linker (residues 195-200) connecting the structurally similar beta-sandwiches of CTD and NTD. The second region is located in a conserved basic patch essential for electrostatic membrane interaction and for triggering assembly of VP40 into the viral matrix to bud nascent virions. The third region is located in the hydrophobic dimeric VP40 CTD-to-CTD interface, which has been shown to be critical for matrix assembly and budding. Residue 307 appears to be particularly important, as the RNA-binding structures formed by the VP40-I307R mutant do not migrate to the membrane, assemble the viral matrix, or bud VLPs. However, the majority of VP40 is located inside the virion and not exposed to the outer surface, a very recent study indicates its importance in inducing immune responses.

Taken together, the invention demonstrates that the humoral responses directed against these three key epitopes of VP40 are useful tools to protect humans against EBOV infection. The protective efficacy of the humoral response could be further evaluated by using functional test such as seroneutralization and/or Antibody-Dependant Cellular Cytotoxicity (ADCC) assays.

The inventors found that serum from most survivors of the three outbreaks, collected a few days after the end of symptoms, reacted mainly with GP peptides. This protein is organized as trimeric spikes consisting of two subunits, namely the extracellular subunit GP₁ attached to the membrane-anchored subunit GP₂. GP₁ is responsible for cell surface attachment, while GP₂ is required for fusion of the viral and host cell membranes. GP is the only viral protein expressed on the surface of infected cells and represents thus an ideal target for neutralizing antibodies.. The ELISA studies showed that reactive GP peptides were distributed in five immunogenic regions located at aa positions 41-67, 93-127, 201-251, 301-359 and 381-411. Two of these regions belong to a domain located in the C-terminal portion of GP₁, which contains a highly N- and O-glycosylated mucin-like domain. This domain is extremely large and represents more than half the molecular weight of GP. Neutralizing antibodies reacting with the mucin-like domain have been shown to protect mice from lethal EBOV infection.

Interestingly, the immunogenic region at aa position 381-411 and the antigenic peptide #118 (aa position 469-483) highlighted in the study covers three linear epitopes (aa positions 401-417, 389-405 and 477-493) recognized by these protective antibodies.

In addition, the above results reveal a new, larger IgG-specific immunodominant region in the mucin-like domain of GP, located at the beginning of the domain protein sequence (aa residues 301 to 359). As GP and sGP share 295 aa in their N-terminal sequences and have several epitopes in common, neutralizing antibodies produced during natural infection appear to react preferentially with sGP, which is abundantly shed by infected cells in acutely infected patients. sGP, appears to act as a decoy for GP-specific neutralizing antibodies. However, the mucin-like domain is specific to GP, suggesting that neutralizing antibodies reacting with this domain would be more effective for the treatment of EBOV infection than those that react with GP and sGP, which would likely be bound by the much more abundant sGP. The crystal structure of the truncated region of this protein suggests that the mucin-like domain extends from the top and sides of GP and effectively dominates its structure, being in a position suitable for host interaction or for protecting the rest of the GP protein from immune recognition. In addition, the heavy glycosylation of this domain could play a role in immune evasion by masking antibody epitopes. Interestingly, a previous study showed that the epitopes of the mucin-like domain recognized by two neutralizing and protective antibodies were not glycosylated. The mucin-like domain does not appear to have a critical function in GP-mediated cell entry or membrane fusion, but it has been implicated in EBOV GP-induced cytotoxicity *in vitro* and has been shown to affect cell adhesion. It has also been reported that this domain is sufficient to cause GP-characteristic cytopathology, while its deletion reduced immunogenicity in a mouse model. Although the sequence of this region is different in the five EBOV species, selective pressure appears to have conserved both its overall length and its level of glycosylation, suggesting an important role in filovirus pathogenesis.

The other three immunogenic regions identified in GP were located in GP₁, being situated on the chalice of the viral surface trimer formed by three GP₁ subunits, that are encircled by three GP₂ subunits forming a cradle. Interestingly, **its was** found that the IgG response encompassed a larger surface area of the chalice bowl in asymptomatic subjects than in survivors.

Two of these three immunogenic regions were located in a putative binding site for attachment to host cells, including residues 54-201, which are sequestered in the chalice bowl of GP₁. The crystal structure of EBOV GP also suggests that the mucin-like domain restricts access to the conserved receptor-binding site. The regions harboring reactive peptides in GP₁ identified here could be relevant for the development of EBOV-protective mAbs.

Interestingly, two immunogenic regions at aa positions 417-439 and 485-515 highlighted in the study cover linear epitopes recognized by mouse mAbs.

In the present study, the peptides reacting with sera from survivors of the three outbreaks were located in similar immunodominant IgG-specific regions, suggesting that the humoral response targets similar epitopes regardless of the EBOV strain.

### Conclusions

The above results show that the humoral response of survivors of Gabonese EBOV outbreaks mainly targeted GP peptides, whereas sera from EBOV-seropositive asymptomatic subjects reacted strongly with VP40 peptides.

The B-cell epitopes identified in the VP35, VP40, NP and GP proteins are relevant for the development of new therapeutics or diagnostic tools and may represent alternatives to EBOV antigens for use in direct ELISA methods.

### References

1. Feldmann H, Klenk HD, Sanchez A (1993) Molecular biology and evolution of filoviruses. Arch Virol Suppl 7: 81-100.
2. Ksiazek T, west Cynthia, Rollin Pierre, Jahrling Peter and Peters C (1999), J of Infectious diseases 179(Suppl 1):192-8.

## Claims

1. An isolated immunogenic peptide, having at least one sequence of 5-35 contiguous aminoacids of an EBOV protein, said at least one sequence being selected in the group comprising sequences SEQ ID N°1 - 61 or being a fragment thereof.

2. The immunogenic peptide of claim 1, having a sequence comprised in protein VP40 of EBOV.

3. The immunogenic peptide of claim 2, comprising one of the sequences SEQ ID N° 1-13.

4. The immunogenic peptide of claim 1, having a sequence comprised in protein GP of EBOV.

5. The immunogenic peptide of claim 4, comprising one of the sequences SEQ ID N° 14-39.

6. The immunogenic peptide of claim 1, having a sequence comprised in protein VP35 of EBOV.

7. The immunogenic peptide of claim 6, comprising one of the sequences SEQ ID N° 40-53.

8. The immunogenic peptide of claim 1, having a sequence comprised in protein NP of EBOV.

9. The immunogenic peptide of claim 8, having one of the sequences SEQ ID N° 54-61.

10. The immunogenic peptide according to anyone of claims 1 to 9, wherein the immunogenic peptide has a sequence corresponding to one of sequences SEQ ID N°1-61.

11. The immunogenic peptide according to anyone of claims 1 to 10, comprising at least 2 sequences from the group consisting of sequences SEQ ID N°1-61, particularly 2-5 sequences from said group.

12. The immunogenic peptide according to anyone of claims 1 to 11, further comprising one or more terminal aminoacid heterologous to EBOV.

13. An immunogenic preparation, comprising a combination of immunopeptides according to anyone of claims 1 to 12.

14. Use of the immunogenic peptide according to anyone of claims 1 to 12 or the immunogenic preparation according to claim 13, to stimulate an immune response directed against EBOVand prepare a vaccinal composition, for the serological detection *in vitro* of EBOV in sera of patients.

15. A kit for the specific diagnosis of EBOLA in a patient, comprising at least one immunogenic peptide such as defined in anyone of claims 1 to 12 or an immunogenic preparation according to claim 13.

16. A method for serological detection of EBOV in a biological sample, comprising
- contacting the biological sample with at least one immunogenic peptide such as defined in anyone of claims 1 to 12 or with an immunogenic preparation such as defined in claim 13,
- detecting the binding of the peptide with antibodies of the sample, for example by ELISA test.


SEQUENCE LISTING

<110>  INSTITUT DE RECHERCHE POUR LE DEVELOPPEMENT (IRD)

<120>  IMMUNOGENIC PEPTIDES OF EBOLA AND APPLICATIONS THEREOF

<130>  63458-4029

<160>  69

<170>  PatentIn version 3.5

<210>  1
<211>  15
<212>  PRT
<213>  Ebola virus

<400>  1

Ala Met Val Asn Val Ile Ser Gly Pro Lys Val Leu Met Lys Gln
1               5                   10                  15


<210>  2
<211>  15
<212>  PRT
<213>  Ebola virus

<400>  2

Ala Asp Gln Lys Thr Tyr Ser Phe Asp Ser Thr Thr Ala Ala Ile
1               5                   10                  15


<210>  3
<211>  15
<212>  PRT
<213>  Ebola virus

<400>  3

His Pro Leu Arg Leu Leu Arg Ile Gly Asn Gln Ala Phe Leu Gln
1               5                   10                  15


<210>  4
<211>  15
<212>  PRT
<213>  Ebola virus

<400>  4

Ala Thr Trp Thr Asp Asp Thr Pro Thr Gly Ser Asn Gly Ala Leu
1               5                   10                  15


<210>  5
<211>  15
<212>  PRT
<213>  Ebola virus

<400>  5

Asp Asp Thr Pro Thr Gly Ser Asn Gly Ala Leu Arg Pro Gly Ile
1               5                   10                  15

<210>  6
<211>  15
<212>  PRT
<213>  Ebola virus

<400>  6

Leu Arg Pro Ile Leu Leu Pro Asn Lys Ser Gly Lys Lys Gly Asn
1               5                   10                  15

<210>  7
<211>  15
<212>  PRT
<213>  Ebola virus

<400>  7

Leu Leu Pro Asn Lys Ser Gly Lys Lys Gly Asn Ser Ala Asp Leu
1               5                   10                  15

<210>  8
<211>  15
<212>  PRT
<213>  Ebola virus

<400>  8

Lys Ser Gly Lys Lys Gly Asn Ser Ala Asp Leu Thr Ser Pro Glu
1               5                   10                  15

<210>  9
<211>  15
<212>  PRT
<213>  Ebola virus

<400>  9

Ile Glu Val Pro Glu Thr Leu Val His Lys Leu Thr Gly Lys Lys
1               5                   10                  15

<210>  10
<211>  15
<212>  PRT
<213>  Ebola virus

<400>  10

Leu Pro Lys Tyr Ile Gly Leu Asp Pro Val Ala Pro Gly Asp Leu
1               5                   10                  15

<210>  11
<211>  15
<212>  PRT
<213>  Ebola virus

<400>  11

Ile Gly Leu Asp Pro Val Ala Pro Gly Asp Leu Thr Met Val Ile
1               5                   10                  15


<210>  12
<211>  15
<212>  PRT
<213>  Ebola virus

<400>  12

Gly Asp Leu Thr Met Val Ile Thr Gln Asp Cys Asp Thr Cys His
1               5                   10                  15


<210>  13
<211>  15
<212>  PRT
<213>  Ebola virus

<400>  13

Met Val Ile Thr Gln Asp Cys Asp Thr Cys His Ser Pro Ala Ser
1               5                   10                  15


<210>  14
<211>  15
<212>  PRT
<213>  Ebola virus

<400>  14

Ser Thr Leu Gln Val Ser Asp Val Asp Lys Leu Val Cys Arg Asp
1               5                   10                  15


<210>  15
<211>  15
<212>  PRT
<213>  Ebola virus

<400>  15

Cys Arg Asp Lys Leu Ser Ser Thr Asn Gln Leu Arg Ser Val Gly
1               5                   10                  15


<210>  16
<211>  15
<212>  PRT
<213>  Ebola virus

<400>  16

Pro Pro Lys Val Val Asn Tyr Glu Ala Gly Glu Trp Ala Glu Asn
1               5                   10                  15


<210>  17

<211>  15
<212>  PRT
<213>  Ebola virus

<400>  17

Ile Lys Lys Pro Asp Gly Ser Glu Cys Leu Pro Ala Ala Pro Asp
1               5                   10                  15


<210>  18
<211>  15
<212>  PRT
<213>  Ebola virus

<400>  18

Glu Pro Val Asn Ala Thr Glu Asp Pro Ser Ser Gly Tyr Tyr Ser
1               5                   10                  15


<210>  19
<211>  15
<212>  PRT
<213>  Ebola virus

<400>  19

Thr Ile Arg Tyr Gln Ala Thr Gly Phe Gly Thr Asn Glu Thr Glu
1               5                   10                  15


<210>  20
<211>  15
<212>  PRT
<213>  Ebola virus

<400>  20

Gln Ala Thr Gly Phe Gly Thr Asn Glu Thr Glu Tyr Leu Phe Glu
1               5                   10                  15


<210>  21
<211>  15
<212>  PRT
<213>  Ebola virus

<400>  21

Phe Gly Thr Asn Glu Thr Glu Tyr Leu Phe Glu Val Asp Asn Leu
1               5                   10                  15


<210>  22
<211>  15
<212>  PRT
<213>  Ebola virus

<400>  22

Asp Asn Leu Thr Tyr Val Gln Leu Glu Ser Arg Phe Thr Pro Gln
1               5                   10                  15

<210>  23
<211>  15
<212>  PRT
<213>  Ebola virus

<400>  23

Leu Ile Trp Lys Val Asn Pro Glu Ile Asp Thr Thr Ile Gly Glu
1               5                   10                  15


<210>  24
<211>  15
<212>  PRT
<213>  Ebola virus

<400>  24

Ile Arg Ser Glu Glu Leu Ser Phe Thr Val Val Ser Asn Gly Ala
1               5                   10                  15


<210>  25
<211>  15
<212>  PRT
<213>  Ebola virus

<400>  25

Thr Val Val Ser Asn Gly Ala Lys Asn Ile Ser Gly Gln Ser Pro
1               5                   10                  15


<210>  26
<211>  15
<212>  PRT
<213>  Ebola virus

<400>  26

Asn Ile Ser Gly Gln Ser Pro Ala Arg Thr Ser Ser Asp Pro Gly
1               5                   10                  15


<210>  27
<211>  15
<212>  PRT
<213>  Ebola virus

<400>  27

Gln Ser Pro Ala Arg Thr Ser Ser Asp Pro Gly Thr Asn Thr Thr
1               5                   10                  15


<210>  28
<211>  15
<212>  PRT
<213>  Ebola virus

<400>  28

Arg Thr Ser Ser Asp Pro Gly Thr Asn Thr Thr Thr Glu Asp His
1               5                   10                  15

<210>  29
<211>  15
<212>  PRT
<213>  Ebola virus

<400>  29

Asp Pro Gly Thr Asn Thr Thr Thr Glu Asp His Lys Ile Met Ala
1               5                   10                  15

<210>  30
<211>  15
<212>  PRT
<213>  Ebola virus

<400>  30

Asn Thr Thr Thr Glu Asp His Lys Ile Met Ala Ser Glu Asn Ser
1               5                   10                  15

<210>  31
<211>  15
<212>  PRT
<213>  Ebola virus

<400>  31

Glu Asp His Lys Ile Met Ala Ser Glu Asn Ser Ser Ala Met Val
1               5                   10                  15

<210>  32
<211>  15
<212>  PRT
<213>  Ebola virus

<400>  32

Ile Met Ala Ser Glu Asn Ser Ser Ala Met Val Gln Val His Ser
1               5                   10                  15

<210>  33
<211>  15
<212>  PRT
<213>  Ebola virus

<400>  33

Glu Asn Ser Ser Ala Met Val Gln Val His Ser Gln Gly Arg Glu
1               5                   10                  15

<210>  34
<211>  15
<212>  PRT

<213>  Ebola virus

<400>  34

Lys Pro Gly Pro Asp Asn Ser Thr His Asn Thr Pro Val Tyr Lys
1               5                   10                  15


<210>  35
<211>  15
<212>  PRT
<213>  Ebola virus

<400>  35

Asp Asn Ser Thr His Asn Thr Pro Val Tyr Lys Leu Asp Ile Ser
1               5                   10                  15


<210>  36
<211>  15
<212>  PRT
<213>  Ebola virus

<400>  36

His Asn Thr Pro Val Tyr Lys Leu Asp Ile Ser Glu Ala Thr Gln
1               5                   10                  15


<210>  37
<211>  15
<212>  PRT
<213>  Ebola virus

<400>  37

Val Tyr Lys Leu Asp Ile Ser Glu Ala Thr Gln Val Glu Gln His
1               5                   10                  15


<210>  38
<211>  15
<212>  PRT
<213>  Ebola virus

<400>  38

Asp Ile Ser Glu Ala Thr Gln Val Glu Gln His His Arg Arg Thr
1               5                   10                  15


<210>  39
<211>  15
<212>  PRT
<213>  Ebola virus

<400>  39

Thr Gly Glu Glu Ser Ala Ser Ser Gly Lys Leu Gly Leu Ile Thr
1               5                   10                  15

<210>  40
<211>  15
<212>  PRT
<213>  Ebola virus

<400>  40

Ala Thr Thr Gln Asn Asp Arg Met Pro Gly Pro Glu Leu Ser Gly
1               5                   10                  15


<210>  41
<211>  15
<212>  PRT
<213>  Ebola virus

<400>  41

Leu Met Thr Gly Arg Ile Pro Val Ser Asp Ile Phe Cys Asp Ile
1               5                   10                  15


<210>  42
<211>  15
<212>  PRT
<213>  Ebola virus

<400>  42

Arg Ile Pro Val Ser Asp Ile Phe Cys Asp Ile Glu Asn Asn Pro
1               5                   10                  15


<210>  43
<211>  15
<212>  PRT
<213>  Ebola virus

<400>  43

Ser Asp Ile Phe Cys Asp Ile Glu Asn Asn Pro Gly Leu Cys Tyr
1               5                   10                  15


<210>  44
<211>  15
<212>  PRT
<213>  Ebola virus

<400>  44

Cys Asp Ile Glu Asn Asn Pro Gly Leu Cys Tyr Ala Ser Gln Met
1               5                   10                  15


<210>  45
<211>  15
<212>  PRT
<213>  Ebola virus

<400>  45

Asn Asn Pro Gly Leu Cys Tyr Ala Ser Gln Met Gln Gln Thr Lys

1               5                   10                  15


<210>  46
<211>  15
<212>  PRT
<213>  Ebola virus

<400>  46

Thr Ala Thr Ala Ala Ala Thr Glu Ala Tyr Trp Ala Glu His Gly
1               5                   10                  15


<210>  47
<211>  15
<212>  PRT
<213>  Ebola virus

<400>  47

Glu His Gly Gln Pro Pro Pro Gly Pro Ser Leu Tyr Glu Glu Ser
1               5                   10                  15


<210>  48
<211>  15
<212>  PRT
<213>  Ebola virus

<400>  48

Ile Glu Ser Arg Asp Glu Thr Val Pro Gln Ser Val Arg Glu Ala
1               5                   10                  15


<210>  49
<211>  15
<212>  PRT
<213>  Ebola virus

<400>  49

Asp Glu Thr Val Pro Gln Ser Val Arg Glu Ala Phe Asn Asn Leu
1               5                   10                  15


<210>  50
<211>  15
<212>  PRT
<213>  Ebola virus

<400>  50

Asp Glu Thr Val Pro Gln Ser Val Arg Glu Ala Phe Asn Asn Leu
1               5                   10                  15


<210>  51
<211>  15
<212>  PRT
<213>  Ebola virus

<400>  51

Ser Thr Thr Ser Leu Thr Glu Glu Asn Phe Gly Lys Pro Asp Ile
1               5                   10                  15


<210>  52
<211>  15
<212>  PRT
<213>  Ebola virus

<400>  52

Leu Thr Glu Glu Asn Phe Gly Lys Pro Asp Ile Ser Ala Lys Asp
1               5                   10                  15


<210>  53
<211>  15
<212>  PRT
<213>  Ebola virus

<400>  53

Val Cys Val Phe Gln Leu Gln Asp Gly Lys Thr Leu Gly Leu Lys
1               5                   10                  15


<210>  54
<211>  15
<212>  PRT
<213>  Ebola virus

<400>  54

Gln Arg Gln Ile Gln Val His Ala Glu Gln Gly Leu Ile Gln Tyr
1               5                   10                  15


<210>  55
<211>  15
<212>  PRT
<213>  Ebola virus

<400>  55

Arg Glu Leu Asp His Leu Gly Leu Asp Asp Gln Glu Lys Lys Ile
1               5                   10                  15


<210>  56
<211>  15
<212>  PRT
<213>  Ebola virus

<400>  56

His Leu Gly Leu Asp Asp Gln Glu Lys Lys Ile Leu Met Asn Phe
1               5                   10                  15


<210>  57
<211>  15

<212>  PRT
<213>  Ebola virus

<400>  57

Gln Lys Lys Asn Glu Ile Ser Phe Gln Gln Thr Asn Ala Met Val
1               5                   10                  15


<210>  58
<211>  15
<212>  PRT
<213>  Ebola virus

<400>  58

Thr Ser Gly His Tyr Asp Asp Asp Asp Asp Ile Pro Phe Pro Gly
1               5                   10                  15


<210>  59
<211>  15
<212>  PRT
<213>  Ebola virus

<400>  59

Asp Asp Ile Pro Phe Pro Gly Pro Ile Asn Asp Asp Asp Asn Pro
1               5                   10                  15


<210>  60
<211>  15
<212>  PRT
<213>  Ebola virus

<400>  60

Leu Phe Asp Leu Asp Glu Asp Asp Glu Asp Thr Lys Pro Val Pro
1               5                   10                  15


<210>  61
<211>  15
<212>  PRT
<213>  Ebola virus

<400>  61

Arg Ser Thr Lys Gly Gly Gln Gln Lys Asn Ser Gln Lys Gly Gln
1               5                   10                  15


<210>  62
<211>  739
<212>  PRT
<213>  Ebola virus

<400>  62

Met Asp Ser Arg Pro Gln Lys Ile Trp Met Ala Pro Ser Leu Thr Gl[?]
1               5                   10                  15

Ser Asp Met Asp Tyr His Lys Ile Leu Thr Ala Gly Leu Ser Val Gl[?]
20 25 30

Gln Gly Ile Val Arg Gln Arg Val Ile Pro Val Tyr Gln Val Asn As[?]
35 40 45

Leu Glu Glu Ile Cys Gln Leu Ile Ile Gln Ala Phe Glu Ala Gly Va[?]
50 55 60

Asp Phe Gln Glu Ser Ala Asp Ser Phe Leu Leu Met Leu Cys Leu Hi[?]
65 70 75 80

His Ala Tyr Gln Gly Asp Tyr Lys Leu Phe Leu Glu Ser Gly Ala Va[?]
85 90 95

Lys Tyr Leu Glu Gly His Gly Phe Arg Phe Glu Val Lys Lys Arg As[?]
100 105 110

Gly Val Lys Arg Leu Glu Glu Leu Leu Pro Ala Val Ser Ser Gly Ly[?]
115 120 125

Asn Ile Lys Arg Thr Leu Ala Ala Met Pro Glu Glu Glu Thr Thr Gl[?]
130 135 140

Ala Asn Ala Gly Gln Phe Leu Ser Phe Ala Ser Leu Phe Leu Pro Ly[?]
145 150 155 16[?]

Leu Val Val Gly Glu Lys Ala Cys Leu Glu Lys Val Gln Arg Gln Il[?]
165 170 175

Gln Val His Ala Glu Gln Gly Leu Ile Gln Tyr Pro Thr Ala Trp Gl[?]
180 185 190

Ser Val Gly His Met Met Val Ile Phe Arg Leu Met Arg Thr Asn Ph[?]
195 200 205

Leu Ile Lys Phe Leu Leu Ile His Gln Gly Met His Met Val Ala Gl[?]
210 215 220

His Asp Ala Asn Asp Ala Val Ile Ser Asn Ser Val Ala Gln Ala Ar[?]
225 230 235 24[?]

Phe Ser Gly Leu Leu Ile Val Lys Thr Val Leu Asp His Ile Leu Gl[?]
245 250 255

Lys Thr Glu Arg Gly Val Arg Leu His Pro Leu Ala Arg Thr Ala Ly[?]
260 265 270

Val Lys Asn Glu Val Asn Ser Phe Lys Ala Ala Leu Ser Ser Leu Ala
        275                 280                 285

Lys His Gly Glu Tyr Ala Pro Phe Ala Arg Leu Leu Asn Leu Ser Gly
    290                 295                 300

Val Asn Asn Leu Glu His Gly Leu Phe Pro Gln Leu Ser Ala Ile Ala
305                 310                 315                 320

Leu Gly Val Ala Thr Ala His Gly Ser Thr Leu Ala Gly Val Asn Val
                325                 330                 335

Gly Glu Gln Tyr Gln Gln Leu Arg Glu Ala Ala Thr Glu Ala Glu Lys
            340                 345                 350

Gln Leu Gln Gln Tyr Ala Glu Ser Arg Glu Leu Asp His Leu Gly Leu
        355                 360                 365

Asp Asp Gln Glu Lys Lys Ile Leu Met Asn Phe His Gln Lys Lys Asn
    370                 375                 380

Glu Ile Ser Phe Gln Gln Thr Asn Ala Met Val Thr Leu Arg Lys Glu
385                 390                 395                 400

Arg Leu Ala Lys Leu Thr Glu Ala Ile Thr Ala Ala Ser Leu Pro Lys
                405                 410                 415

Thr Ser Gly His Tyr Asp Asp Asp Asp Asp Ile Pro Phe Pro Gly Pro
            420                 425                 430

Ile Asn Asp Asp Asp Asn Pro Gly His Gln Asp Asp Asp Pro Thr Asp
        435                 440                 445

Ser Gln Asp Thr Thr Ile Pro Asp Val Val Val Asp Pro Asp Asp Gly
    450                 455                 460

Ser Tyr Gly Glu Tyr Gln Ser Tyr Ser Glu Asn Gly Met Asn Ala Pro
465                 470                 475                 480

Asp Asp Leu Val Leu Phe Asp Leu Asp Glu Asp Asp Glu Asp Thr Lys
                485                 490                 495

Pro Val Pro Asn Arg Ser Thr Lys Gly Gly Gln Gln Lys Asn Ser Gln
            500                 505                 510

Lys Gly Gln His Ile Glu Gly Arg Gln Thr Gln Ser Arg Pro Ile Gln

515                 520                 525

Asn Val Pro Gly Pro His Arg Thr Ile His His Ala Ser Ala Pro Leu
    530                 535                 540

Thr Asp Asn Asp Arg Arg Asn Glu Pro Ser Gly Ser Thr Ser Pro Arg
545                 550                 555                 560

Met Leu Thr Pro Ile Asn Glu Glu Ala Asp Pro Leu Asp Asp Ala Asp
                565                 570                 575

Asp Glu Thr Ser Ser Leu Pro Pro Leu Glu Ser Asp Asp Glu Glu Gln
            580                 585                 590

Asp Arg Asp Gly Thr Ser Asn Arg Thr Pro Thr Val Ala Pro Pro Ala
        595                 600                 605

Pro Val Tyr Arg Asp His Ser Glu Lys Lys Glu Leu Pro Gln Asp Glu
    610                 615                 620

Gln Gln Asp Gln Asp His Thr Gln Glu Ala Arg Asn Gln Asp Ser Asp
625                 630                 635                 640

Asn Thr Gln Ser Glu His Ser Phe Glu Glu Met Tyr Arg His Ile Leu
                645                 650                 655

Arg Ser Gln Gly Pro Phe Asp Ala Val Leu Tyr Tyr His Met Met Lys
            660                 665                 670

Asp Glu Pro Val Val Phe Ser Thr Ser Asp Gly Lys Glu Tyr Thr Tyr
        675                 680                 685

Pro Asp Ser Leu Glu Glu Glu Tyr Pro Pro Trp Leu Thr Glu Lys Glu
    690                 695                 700

Ala Met Asn Glu Glu Asn Arg Phe Val Thr Leu Asp Gly Gln Gln Phe
705                 710                 715                 720

Tyr Trp Pro Val Met Asn His Lys Asn Lys Phe Met Ala Ile Leu Gln
                725                 730                 735

His His Gln

<210>  63
<211>  739
<212>  PRT
<213>  Ebola virus

<400> 63

Met Asp Ser Arg Pro Gln Lys Ile Trp Met Ala Pro Ser Leu Thr Glu
1               5                   10                  15

Ser Asp Met Asp Tyr His Lys Ile Leu Thr Ala Gly Leu Ser Val Gln
            20                  25                  30

Gln Gly Ile Val Arg Gln Arg Val Ile Pro Val Tyr Gln Val Asn Asn
        35                  40                  45

Leu Glu Glu Ile Cys Gln Leu Ile Ile Gln Ala Phe Glu Ala Gly Val
    50                  55                  60

Asp Phe Gln Glu Ser Ala Asp Ser Phe Leu Leu Met Leu Cys Leu His
65                  70                  75                  80

His Ala Tyr Gln Gly Asp Tyr Lys Leu Phe Leu Glu Ser Gly Ala Val
                85                  90                  95

Lys Tyr Leu Glu Gly His Gly Phe Arg Phe Glu Val Lys Lys Arg Asp
            100                 105                 110

Gly Val Lys Arg Leu Glu Glu Leu Leu Pro Ala Val Ser Ser Gly Lys
        115                 120                 125

Asn Ile Lys Arg Thr Leu Ala Ala Met Pro Glu Glu Glu Thr Thr Glu
    130                 135                 140

Ala Asn Ala Gly Gln Phe Leu Ser Phe Ala Ser Leu Phe Leu Pro Lys
145                 150                 155                 160

Leu Val Val Gly Glu Lys Ala Cys Leu Glu Lys Val Gln Arg Gln Ile
                165                 170                 175

Gln Val His Ala Glu Gln Gly Leu Ile Gln Tyr Pro Thr Ala Trp Gln
            180                 185                 190

Ser Val Gly His Met Met Val Ile Phe Arg Leu Met Arg Thr Asn Phe
        195                 200                 205

Leu Ile Lys Phe Leu Leu Ile His Gln Gly Met His Met Val Ala Gly
    210                 215                 220

His Asp Ala Asn Asp Ala Val Ile Ser Asn Ser Val Ala Gln Ala Arg
225                 230                 235                 240

Phe Ser Gly Leu Leu Ile Val Lys Thr Val Leu Asp His Ile Leu Gln
                    245                 250                 255

Lys Thr Glu Arg Gly Val Arg Leu His Pro Leu Ala Arg Thr Ala Lys
            260                 265                 270

Val Lys Asn Glu Val Asn Ser Phe Lys Ala Ala Leu Ser Ser Leu Ala
        275                 280                 285

Lys His Gly Glu Tyr Ala Pro Phe Ala Arg Leu Leu Asn Leu Ser Gly
    290                 295                 300

Val Asn Asn Leu Glu His Gly Leu Phe Pro Gln Leu Ser Ala Ile Ala
305                 310                 315                 320

Leu Gly Val Ala Thr Ala His Gly Ser Thr Leu Ala Gly Val Asn Val
                    325                 330                 335

Gly Glu Gln Tyr Gln Gln Leu Arg Glu Ala Ala Thr Glu Ala Glu Lys
            340                 345                 350

Gln Leu Gln Gln Tyr Ala Glu Ser Arg Glu Leu Asp His Leu Gly Leu
        355                 360                 365

Asp Asp Gln Glu Lys Lys Ile Leu Met Asn Phe His Gln Lys Lys Asn
    370                 375                 380

Glu Ile Ser Phe Gln Gln Thr Asn Ala Met Val Thr Leu Arg Lys Glu
385                 390                 395                 400

Arg Leu Ala Lys Leu Thr Glu Ala Ile Thr Ala Ala Ser Leu Pro Lys
                    405                 410                 415

Thr Ser Gly His Tyr Asp Asp Asp Asp Asp Ile Pro Phe Pro Gly Pro
            420                 425                 430

Ile Asn Asp Asp Asp Asn Pro Gly His Gln Asp Asp Asp Pro Thr Asp
        435                 440                 445

Ser Gln Asp Thr Thr Ile Pro Asp Val Val Val Asp Pro Asp Asp Gly
    450                 455                 460

Ser Tyr Gly Glu Tyr Gln Ser Tyr Ser Glu Asn Gly Met Asn Ala Pro
465                 470                 475                 480

Asp Asp Leu Val Leu Phe Asp Leu Asp Glu Asp Asp Glu Asp Thr Lys
                    485                 490                 495

Pro Val Pro Asn Arg Ser Thr Lys Gly Gly Gln Gln Lys Asn Ser Gln
        500                 505                 510

Lys Gly Gln His Ile Glu Gly Arg Gln Thr Gln Ser Arg Pro Ile Gln
    515                 520                 525

Asn Val Pro Gly Pro His Arg Thr Ile His His Ala Ser Ala Pro Leu
    530                 535                 540

Thr Asp Asn Asp Arg Arg Asn Glu Pro Ser Gly Ser Thr Ser Pro Arg
545                 550                 555                 560

Met Leu Thr Pro Ile Asn Glu Glu Ala Asp Pro Leu Asp Asp Ala Asp
                565                 570                 575

Asp Glu Thr Ser Ser Leu Pro Pro Leu Glu Ser Asp Asp Glu Glu Gln
        580                 585                 590

Asp Arg Asp Gly Thr Ser Asn Arg Thr Pro Thr Val Ala Pro Pro Ala
        595                 600                 605

Pro Val Tyr Arg Asp His Ser Glu Lys Lys Glu Leu Pro Gln Asp Glu
    610                 615                 620

Gln Gln Asp Gln Asp His Thr Gln Glu Ala Arg Asn Gln Asp Ser Asp
625                 630                 635                 640

Asn Thr Gln Ser Glu His Ser Phe Glu Glu Met Tyr Arg His Ile Leu
                645                 650                 655

Arg Ser Gln Gly Pro Phe Asp Ala Val Leu Tyr Tyr His Met Met Lys
        660                 665                 670

Asp Glu Pro Val Val Phe Ser Thr Ser Asp Gly Lys Glu Tyr Thr Tyr
        675                 680                 685

Pro Asp Ser Leu Glu Glu Glu Tyr Pro Pro Trp Leu Thr Glu Lys Glu
    690                 695                 700

Ala Met Asn Glu Glu Asn Arg Phe Val Thr Leu Asp Gly Gln Gln Phe
705                 710                 715                 720

Tyr Trp Pro Val Met Asn His Lys Asn Lys Phe Met Ala Ile Leu Gln
                725                 730                 735

His His Gln

<210>  64
<211>  340
<212>  PRT
<213>  Ebola virus

<400>  64

Met Thr Thr Arg Thr Lys Gly Arg Gly His Thr Ala Ala Thr Thr Gln
1               5                   10                  15

Asn Asp Arg Met Pro Gly Pro Glu Leu Ser Gly Trp Ile Ser Glu Gln
            20                  25                  30

Leu Met Thr Gly Arg Ile Pro Val Ser Asp Ile Phe Cys Asp Ile Glu
        35                  40                  45

Asn Asn Pro Gly Leu Cys Tyr Ala Ser Gln Met Gln Gln Thr Lys Pro
    50                  55                  60

Asn Pro Lys Thr Arg Asn Ser Gln Thr Gln Thr Asp Pro Ile Cys Asn
65                  70                  75                  80

His Ser Phe Glu Glu Val Val Gln Thr Leu Ala Ser Leu Ala Thr Val
                85                  90                  95

Val Gln Gln Gln Thr Ile Ala Ser Glu Ser Leu Glu Gln Arg Ile Thr
            100                 105                 110

Ser Leu Glu Asn Gly Leu Lys Pro Val Tyr Asp Met Ala Lys Thr Ile
        115                 120                 125

Ser Ser Leu Asn Arg Val Cys Ala Glu Met Val Ala Lys Tyr Asp Leu
    130                 135                 140

Leu Val Met Thr Thr Gly Arg Ala Thr Ala Thr Ala Ala Ala Thr Glu
145                 150                 155                 160

Ala Tyr Trp Ala Glu His Gly Gln Pro Pro Pro Gly Pro Ser Leu Tyr
                165                 170                 175

Glu Glu Ser Ala Ile Arg Gly Lys Ile Glu Ser Arg Asp Glu Thr Val
            180                 185                 190

Pro Gln Ser Val Arg Glu Ala Phe Asn Asn Leu Asn Ser Thr Thr Ser
        195                 200                 205

Leu Thr Glu Glu Asn Phe Gly Lys Pro Asp Ile Ser Ala Lys Asp Leu
    210                 215                 220

Arg Asn Ile Met Tyr Asp His Leu Pro Gly Phe Gly Thr Ala Phe His
225                 230                 235                 240

Gln Leu Val Gln Val Ile Cys Lys Leu Gly Lys Asp Ser Asn Ser Leu
                245                 250                 255

Asp Ile Ile His Ala Glu Phe Gln Ala Ser Leu Ala Glu Gly Asp Ser
            260                 265                 270

Pro Gln Cys Ala Leu Ile Gln Ile Thr Lys Arg Val Pro Ile Phe Gln
        275                 280                 285

Asp Ala Ala Pro Pro Val Ile His Ile Arg Ser Arg Gly Asp Ile Pro
    290                 295                 300

Arg Ala Cys Gln Lys Ser Leu Arg Pro Val Pro Pro Ser Pro Lys Ile
305                 310                 315                 320

Asp Arg Gly Trp Val Cys Val Phe Gln Leu Gln Asp Gly Lys Thr Leu
                325                 330                 335

Gly Leu Lys Ile
            340

<210>  65
<211>  340
<212>  PRT
<213>  Ebola virus

<400>  65

Met Thr Thr Arg Thr Lys Gly Arg Gly His Thr Ala Ala Thr Thr Gln
1               5                   10                  15

Asn Asp Arg Met Pro Gly Pro Glu Leu Ser Gly Trp Ile Ser Glu Gln
            20                  25                  30

Leu Met Thr Gly Arg Ile Pro Val Ser Asp Ile Phe Cys Asp Ile Glu
        35                  40                  45

Asn Asn Pro Gly Leu Cys Tyr Ala Ser Gln Met Gln Gln Thr Lys Pro
    50                  55                  60

Asn Pro Lys Thr Arg Asn Ser Gln Thr Gln Thr Asp Pro Ile Cys Asn
65                  70                  75                  80

His Ser Phe Glu Glu Val Val Gln Thr Leu Ala Ser Leu Ala Thr Val
                85                  90                  95

Val Gln Gln Gln Thr Ile Ala Ser Glu Ser Leu Glu Gln Arg Ile Thr
            100                 105                 110

Ser Leu Glu Asn Gly Leu Lys Pro Val Tyr Asp Met Ala Lys Thr Ile
        115                 120                 125

Ser Ser Leu Asn Arg Val Cys Ala Glu Met Val Ala Lys Tyr Asp Leu
    130                 135                 140

Leu Val Met Thr Thr Gly Arg Ala Thr Ala Thr Ala Ala Ala Thr Glu
145                 150                 155                 160

Ala Tyr Trp Ala Glu His Gly Gln Pro Pro Pro Gly Pro Ser Leu Tyr
                165                 170                 175

Glu Glu Ser Ala Ile Arg Gly Lys Ile Glu Ser Arg Asp Glu Thr Val
            180                 185                 190

Pro Gln Ser Val Arg Glu Ala Phe Asn Asn Leu Asn Ser Thr Thr Ser
        195                 200                 205

Leu Thr Glu Glu Asn Phe Gly Lys Pro Asp Ile Ser Ala Lys Asp Leu
    210                 215                 220

Arg Asn Ile Met Tyr Asp His Leu Pro Gly Phe Gly Thr Ala Phe His
225                 230                 235                 240

Gln Leu Val Gln Val Ile Cys Lys Leu Gly Lys Asp Ser Asn Ser Leu
                245                 250                 255

Asp Ile Ile His Ala Glu Phe Gln Ala Ser Leu Ala Glu Gly Asp Ser
            260                 265                 270

Pro Gln Cys Ala Leu Ile Gln Ile Thr Lys Arg Val Pro Ile Phe Gln
        275                 280                 285

Asp Ala Ala Pro Pro Val Ile His Ile Arg Ser Arg Gly Asp Ile Pro
    290                 295                 300

Arg Ala Cys Gln Lys Ser Leu Arg Pro Val Pro Pro Ser Pro Lys Ile
305                 310                 315                 320

Asp Arg Gly Trp Val Cys Val Phe Gln Leu Gln Asp Gly Lys Thr Leu
                325                 330                 335

Gly Leu Lys Ile

            340

<210>  66
<211>  676
<212>  PRT
<213>  Ebola virus

<400>  66

Met Gly Val Thr Gly Ile Leu Gln Leu Pro Arg Asp Arg Phe Lys Arg
1               5                   10                  15

Thr Ser Phe Phe Leu Trp Val Ile Ile Leu Phe Gln Arg Thr Phe Ser
            20                  25                  30

Ile Pro Leu Gly Val Ile His Asn Ser Thr Leu Gln Val Ser Asp Val
        35                  40                  45

Asp Lys Leu Val Cys Arg Asp Lys Leu Ser Ser Thr Asn Gln Leu Arg
    50                  55                  60

Ser Val Gly Leu Asn Leu Glu Gly Asn Gly Val Ala Thr Asp Val Pro
65                  70                  75                  80

Ser Ala Thr Lys Arg Trp Gly Phe Arg Ser Gly Val Pro Pro Lys Val
                85                  90                  95

Val Asn Tyr Glu Ala Gly Glu Trp Ala Glu Asn Cys Tyr Asn Leu Glu
            100                 105                 110

Ile Lys Lys Pro Asp Gly Ser Glu Cys Leu Pro Ala Ala Pro Asp Gly
        115                 120                 125

Ile Arg Gly Phe Pro Arg Cys Arg Tyr Val His Lys Val Ser Gly Thr
    130                 135                 140

Gly Pro Cys Ala Gly Asp Phe Ala Phe His Lys Glu Gly Ala Phe Phe
145                 150                 155                 160

Leu Tyr Asp Arg Leu Ala Ser Thr Val Ile Tyr Arg Gly Thr Thr Phe
                165                 170                 175

Ala Glu Gly Val Val Ala Phe Leu Ile Leu Pro Gln Ala Lys Lys Asp
            180                 185                 190

Phe Phe Ser Ser His Pro Leu Arg Glu Pro Val Asn Ala Thr Glu Asp
        195                 200                 205

Pro Ser Ser Gly Tyr Tyr Ser Thr Thr Ile Arg Tyr Gln Ala Thr Gly

210                 215                 220

Phe Gly Thr Asn Glu Thr Glu Tyr Leu Phe Glu Val Asp Asn Leu Thr
225                 230                 235                 240

Tyr Val Gln Leu Glu Ser Arg Phe Thr Pro Gln Phe Leu Leu Gln Leu
                245                 250                 255

Asn Glu Thr Ile Tyr Thr Ser Gly Lys Arg Ser Asn Thr Thr Gly Lys
            260                 265                 270

Leu Ile Trp Lys Val Asn Pro Glu Ile Asp Thr Thr Ile Gly Glu Trp
        275                 280                 285

Ala Phe Trp Glu Thr Lys Lys Asn Leu Thr Arg Lys Ile Arg Ser Glu
    290                 295                 300

Glu Leu Ser Phe Thr Val Val Ser Asn Gly Ala Lys Asn Ile Ser Gly
305                 310                 315                 320

Gln Ser Pro Ala Arg Thr Ser Ser Asp Pro Gly Thr Asn Thr Thr Thr
                325                 330                 335

Glu Asp His Lys Ile Met Ala Ser Glu Asn Ser Ser Ala Met Val Gln
            340                 345                 350

Val His Ser Gln Gly Arg Glu Ala Ala Val Ser His Leu Thr Thr Leu
        355                 360                 365

Ala Thr Ile Ser Thr Ser Pro Gln Ser Leu Thr Thr Lys Pro Gly Pro
    370                 375                 380

Asp Asn Ser Thr His Asn Thr Pro Val Tyr Lys Leu Asp Ile Ser Glu
385                 390                 395                 400

Ala Thr Gln Val Glu Gln His His Arg Arg Thr Asp Asn Asp Ser Thr
                405                 410                 415

Ala Ser Asp Thr Pro Ser Ala Thr Thr Ala Ala Gly Pro Pro Lys Ala
            420                 425                 430

Glu Asn Thr Asn Thr Ser Lys Ser Thr Asp Phe Leu Asp Pro Ala Thr
        435                 440                 445

Thr Thr Ser Pro Gln Asn His Ser Glu Thr Ala Gly Asn Asn Asn Thr
    450                 455                 460

His His Gln Asp Thr Gly Glu Glu Ser Ala Ser Ser Gly Lys Leu Gly
465                 470                 475                 480

Leu Ile Thr Asn Thr Ile Ala Gly Val Ala Gly Leu Ile Thr Gly Gly
                485                 490                 495

Arg Arg Thr Arg Arg Glu Ala Ile Val Asn Ala Gln Pro Lys Cys Asn
            500                 505                 510

Pro Asn Leu His Tyr Trp Thr Thr Gln Asp Glu Gly Ala Ala Ile Gly
        515                 520                 525

Leu Ala Trp Ile Pro Tyr Phe Gly Pro Ala Ala Glu Gly Ile Tyr Ile
    530                 535                 540

Glu Gly Leu Met His Asn Gln Asp Gly Leu Ile Cys Gly Leu Arg Gln
545                 550                 555                 560

Leu Ala Asn Glu Thr Thr Gln Ala Leu Gln Leu Phe Leu Arg Ala Thr
                565                 570                 575

Thr Glu Leu Arg Thr Phe Ser Ile Leu Asn Arg Lys Ala Ile Asp Phe
            580                 585                 590

Leu Leu Gln Arg Trp Gly Gly Thr Cys His Ile Leu Gly Pro Asp Cys
        595                 600                 605

Cys Ile Glu Pro His Asp Trp Thr Lys Asn Ile Thr Asp Lys Ile Asp
    610                 615                 620

Gln Ile Ile His Asp Phe Val Asp Lys Thr Leu Pro Asp Gln Gly Asp
625                 630                 635                 640

Asn Asp Asn Trp Trp Thr Gly Trp Arg Gln Trp Ile Pro Ala Gly Ile
                645                 650                 655

Gly Val Thr Gly Val Ile Ile Ala Val Ile Ala Leu Phe Cys Ile Cys
            660                 665                 670

Lys Phe Val Phe
        675

<210>  67
<211>  676
<212>  PRT
<213>  Ebola virus

<400>  67

Met Gly Val Thr Gly Ile Leu Gln Leu Pro Arg Asp Arg Phe Lys Arg
1               5                   10                  15

Thr Ser Phe Phe Leu Trp Val Ile Ile Leu Phe Gln Arg Thr Phe Ser
            20                  25                  30

Ile Pro Leu Gly Val Ile His Asn Ser Thr Leu Gln Val Ser Asp Val
        35                  40                  45

Asp Lys Leu Val Cys Arg Asp Lys Leu Ser Ser Thr Asn Gln Leu Arg
    50                  55                  60

Ser Val Gly Leu Asn Leu Glu Gly Asn Gly Val Ala Thr Asp Val Pro
65                  70                  75                  80

Ser Ala Thr Lys Arg Trp Gly Phe Arg Ser Gly Val Pro Pro Lys Val
                85                  90                  95

Val Asn Tyr Glu Ala Gly Glu Trp Ala Glu Asn Cys Tyr Asn Leu Glu
            100                 105                 110

Ile Lys Lys Pro Asp Gly Ser Glu Cys Leu Pro Ala Ala Pro Asp Gly
        115                 120                 125

Ile Arg Gly Phe Pro Arg Cys Arg Tyr Val His Lys Val Ser Gly Thr
    130                 135                 140

Gly Pro Cys Ala Gly Asp Phe Ala Phe His Lys Glu Gly Ala Phe Phe
145                 150                 155                 160

Leu Tyr Asp Arg Leu Ala Ser Thr Val Ile Tyr Arg Gly Thr Thr Phe
                165                 170                 175

Ala Glu Gly Val Val Ala Phe Leu Ile Leu Pro Gln Ala Lys Lys Asp
            180                 185                 190

Phe Phe Ser Ser His Pro Leu Arg Glu Pro Val Asn Ala Thr Glu Asp
        195                 200                 205

Pro Ser Ser Gly Tyr Tyr Ser Thr Thr Ile Arg Tyr Gln Ala Thr Gly
    210                 215                 220

Phe Gly Thr Asn Glu Thr Glu Tyr Leu Phe Glu Val Asp Asn Leu Thr
225                 230                 235                 240

Tyr Val Gln Leu Glu Ser Arg Phe Thr Pro Gln Phe Leu Leu Gln Leu
                245                 250                 255

Asn Glu Thr Ile Tyr Thr Ser Gly Lys Arg Ser Asn Thr Thr Gly Lys
        260                 265                 270

Leu Ile Trp Lys Val Asn Pro Glu Ile Asp Thr Thr Ile Gly Glu Trp
    275                 280                 285

Ala Phe Trp Glu Thr Lys Lys Asn Leu Thr Arg Lys Ile Arg Ser Glu
    290                 295                 300

Glu Leu Ser Phe Thr Val Val Ser Asn Gly Ala Lys Asn Ile Ser Gly
305                 310                 315                 320

Gln Ser Pro Ala Arg Thr Ser Ser Asp Pro Gly Thr Asn Thr Thr Thr
                325                 330                 335

Glu Asp His Lys Ile Met Ala Ser Glu Asn Ser Ser Ala Met Val Gln
            340                 345                 350

Val His Ser Gln Gly Arg Glu Ala Ala Val Ser His Leu Thr Thr Leu
        355                 360                 365

Ala Thr Ile Ser Thr Ser Pro Gln Ser Leu Thr Thr Lys Pro Gly Pro
    370                 375                 380

Asp Asn Ser Thr His Asn Thr Pro Val Tyr Lys Leu Asp Ile Ser Glu
385                 390                 395                 400

Ala Thr Gln Val Glu Gln His His Arg Arg Thr Asp Asn Asp Ser Thr
                405                 410                 415

Ala Ser Asp Thr Pro Ser Ala Thr Thr Ala Ala Gly Pro Pro Lys Ala
            420                 425                 430

Glu Asn Thr Asn Thr Ser Lys Ser Thr Asp Phe Leu Asp Pro Ala Thr
        435                 440                 445

Thr Thr Ser Pro Gln Asn His Ser Glu Thr Ala Gly Asn Asn Asn Thr
    450                 455                 460

His His Gln Asp Thr Gly Glu Glu Ser Ala Ser Ser Gly Lys Leu Gly
465                 470                 475                 480

Leu Ile Thr Asn Thr Ile Ala Gly Val Ala Gly Leu Ile Thr Gly Gly
                485                 490                 495

Arg Arg Thr Arg Arg Glu Ala Ile Val Asn Ala Gln Pro Lys Cys Asn
            500                 505                 510

Pro Asn Leu His Tyr Trp Thr Thr Gln Asp Glu Gly Ala Ala Ile Gly
        515                 520                 525

Leu Ala Trp Ile Pro Tyr Phe Gly Pro Ala Ala Glu Gly Ile Tyr Ile
    530                 535                 540

Glu Gly Leu Met His Asn Gln Asp Gly Leu Ile Cys Gly Leu Arg Gln
545                 550                 555                 560

Leu Ala Asn Glu Thr Thr Gln Ala Leu Gln Leu Phe Leu Arg Ala Thr
                565                 570                 575

Thr Glu Leu Arg Thr Phe Ser Ile Leu Asn Arg Lys Ala Ile Asp Phe
            580                 585                 590

Leu Leu Gln Arg Trp Gly Gly Thr Cys His Ile Leu Gly Pro Asp Cys
        595                 600                 605

Cys Ile Glu Pro His Asp Trp Thr Lys Asn Ile Thr Asp Lys Ile Asp
    610                 615                 620

Gln Ile Ile His Asp Phe Val Asp Lys Thr Leu Pro Asp Gln Gly Asp
625                 630                 635                 640

Asn Asp Asn Trp Trp Thr Gly Trp Arg Gln Trp Ile Pro Ala Gly Ile
                645                 650                 655

Gly Val Thr Gly Val Ile Ile Ala Val Ile Ala Leu Phe Cys Ile Cys
            660                 665                 670

Lys Phe Val Phe
        675

<210>  68
<211>  326
<212>  PRT
<213>  Ebola virus

<400>  68

Met Arg Arg Val Ile Leu Pro Thr Ala Pro Pro Glu Tyr Met Glu Ala
1               5                   10                  15

Ile Tyr Pro Val Arg Ser Asn Ser Thr Ile Ala Arg Gly Gly Asn Ser
            20                  25                  30

Asn Thr Gly Phe Leu Thr Pro Glu Ser Val Asn Gly Asp Thr Pro Ser
        35                  40                  45

Asn Pro Leu Arg Pro Ile Ala Asp Asp Thr Ile Asp His Ala Ser His
    50                  55                  60

Thr Pro Gly Ser Val Ser Ser Ala Phe Ile Leu Glu Ala Met Val Asn
65                  70                  75                  80

Val Ile Ser Gly Pro Lys Val Leu Met Lys Gln Ile Pro Ile Trp Leu
                85                  90                  95

Pro Leu Gly Val Ala Asp Gln Lys Thr Tyr Ser Phe Asp Ser Thr Thr
            100                 105                 110

Ala Ala Ile Met Leu Ala Ser Tyr Thr Ile Thr His Phe Gly Lys Ala
        115                 120                 125

Thr Asn Pro Leu Val Arg Val Asn Arg Leu Gly Pro Gly Ile Pro Asp
    130                 135                 140

His Pro Leu Arg Leu Leu Arg Ile Gly Asn Gln Ala Phe Leu Gln Glu
145                 150                 155                 160

Phe Val Leu Pro Pro Val Gln Leu Pro Gln Tyr Phe Thr Phe Asp Leu
                165                 170                 175

Thr Ala Leu Lys Leu Ile Thr Gln Pro Leu Pro Ala Ala Thr Trp Thr
            180                 185                 190

Asp Asp Thr Pro Thr Gly Ser Asn Gly Ala Leu Arg Pro Gly Ile Ser
        195                 200                 205

Phe His Pro Lys Leu Arg Pro Ile Leu Leu Pro Asn Lys Ser Gly Lys
    210                 215                 220

Lys Gly Asn Ser Ala Asp Leu Thr Ser Pro Glu Lys Ile Gln Ala Ile
225                 230                 235                 240

Met Thr Ser Leu Gln Asp Phe Lys Ile Val Pro Ile Asp Pro Thr Lys
                245                 250                 255

Asn Ile Met Gly Ile Glu Val Pro Glu Thr Leu Val His Lys Leu Thr
            260                 265                 270

Gly Lys Lys Val Thr Ser Lys Asn Gly Gln Pro Ile Ile Pro Val Leu
        275                 280                 285

Leu Pro Lys Tyr Ile Gly Leu Asp Pro Val Ala Pro Gly Asp Leu Thr

290                 295                 300

Met Val Ile Thr Gln Asp Cys Asp Thr Cys His Ser Pro Ala Ser Leu
305                 310                 315                 320

Pro Ala Val Ile Glu Lys
                325

<210>  69
<211>  326
<212>  PRT
<213>  Ebola virus

<400>  69

Met Arg Arg Val Ile Leu Pro Thr Ala Pro Pro Glu Tyr Met Glu Ala
1               5                   10                  15

Ile Tyr Pro Val Arg Ser Asn Ser Thr Ile Ala Arg Gly Gly Asn Ser
            20                  25                  30

Asn Thr Gly Phe Leu Thr Pro Glu Ser Val Asn Gly Asp Thr Pro Ser
        35                  40                  45

Asn Pro Leu Arg Pro Ile Ala Asp Asp Thr Ile Asp His Ala Ser His
    50                  55                  60

Thr Pro Gly Ser Val Ser Ser Ala Phe Ile Leu Glu Ala Met Val Asn
65                  70                  75                  80

Val Ile Ser Gly Pro Lys Val Leu Met Lys Gln Ile Pro Ile Trp Leu
                85                  90                  95

Pro Leu Gly Val Ala Asp Gln Lys Thr Tyr Ser Phe Asp Ser Thr Thr
            100                 105                 110

Ala Ala Ile Met Leu Ala Ser Tyr Thr Ile Thr His Phe Gly Lys Ala
        115                 120                 125

Thr Asn Pro Leu Val Arg Val Asn Arg Leu Gly Pro Gly Ile Pro Asp
    130                 135                 140

His Pro Leu Arg Leu Leu Arg Ile Gly Asn Gln Ala Phe Leu Gln Glu
145                 150                 155                 160

Phe Val Leu Pro Pro Val Gln Leu Pro Gln Tyr Phe Thr Phe Asp Leu
                165                 170                 175

Thr Ala Leu Lys Leu Ile Thr Gln Pro Leu Pro Ala Ala Thr Trp Thr

            180                 185                 190

Asp Asp Thr Pro Thr Gly Ser Asn Gly Ala Leu Arg Pro Gly Ile Ser
        195                 200                 205

Phe His Pro Lys Leu Arg Pro Ile Leu Leu Pro Asn Lys Ser Gly Lys
    210                 215                 220

Lys Gly Asn Ser Ala Asp Leu Thr Ser Pro Glu Lys Ile Gln Ala Ile
225                 230                 235                 240

Met Thr Ser Leu Gln Asp Phe Lys Ile Val Pro Ile Asp Pro Thr Lys
                245                 250                 255

Asn Ile Met Gly Ile Glu Val Pro Glu Thr Leu Val His Lys Leu Thr
            260                 265                 270

Gly Lys Lys Val Thr Ser Lys Asn Gly Gln Pro Ile Ile Pro Val Leu
        275                 280                 285

Leu Pro Lys Tyr Ile Gly Leu Asp Pro Val Ala Pro Gly Asp Leu Thr
    290                 295                 300

Met Val Ile Thr Gln Asp Cys Asp Thr Cys His Ser Pro Ala Ser Leu
305                 310                 315                 320

Pro Ala Val Ile Glu Lys
                325